# EUROPEAN PATENT SPECIFICATION

(11) **EP 4 176 901 B1**
(45) Date of publication and mention of the grant of the patent: **04.10.2023**
(21) Application number: 21213698.0
(22) Date of filing: 10.12.2021
(51) Int. Cl.: A61K 38/08, C07K 7/06, A61K 9/19, A61K 47/18

(54) **STABLE COMPOSITIONS OF FOXY-5 HEXAPEPTIDE WITH HIGH SOLUBILITY COMPRISING A NITROGEN BASE**
STABILE ZUSAMMENSETZUNGEN VON FOXO-5-HEXAPEPTID MIT HOHER LÖSLICHKEIT ENTHALTEND EINE STICKSTOFFBASE
COMPOSITIONS STABLES D'HEXAPEPTIDE FOXY-5 À FORTE SOLUBILITÉ COMPRENANT UNE BASE AZOTÉE

(43) Date of publication of application: 10.05.2023
(73) Proprietor: Wntresearch AB, 222 25 Malmö (SE)
(72) Inventor: Henriksen, Dennis, 205 12 Malmö (SE)
(74) Representative: AWA Denmark A/S

(56) References cited:
- WO-A1-2006/130082
- WO-A1-2019/201899
- WO-A1-2020/038878
- Anonymous: "Product Name: Foxy 5 , certificate of analysis", Tocris , Product sheet, 21 March 2021 (2021-03-21), XP055919742, Retrieved from the Internet: URL:https://resources.rndsystems.com/pdfs/ tocris_coa/5461_5_coa.pdf?t=1652191762&_ga =2.25925694.247523588.1652191764-183743357 7.1652191764 [retrieved on 2022-05-10]
- Anonymous: "WntResearch AB (publ) 556738-7864 WntResearch receives positive opinion from the European Patent Office, EPO, regarding Foxy-5 manufacturing", WntResearch, 10 July 2020 (2020-07-10), XP055919501, Retrieved from the Internet: URL:https://mb.cision.com/Main/11610/31521 95/1277643.pdf [retrieved on 2022-05-10]

## Description

### FIELD OF THE INVENTION

The present disclosure relates to new, highly soluble compositions of the Wnt hexapeptide Foxy-5. The present disclosure further relates to methods for the production of said compositions and stability assessments thereof.

### BACKGROUND OF THE INVENTION

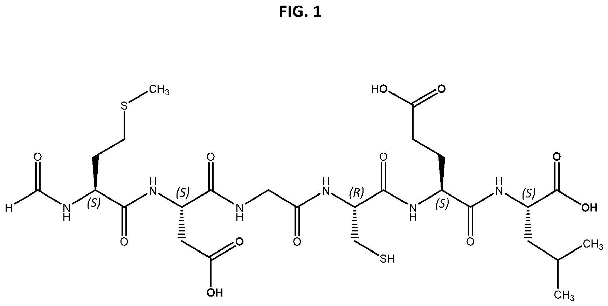

Foxy-5 is a formylated, WNT5A-derived hexapeptide and WNT5A mimetic with potential anti-metastatic activity currently in development as a drug candidate for the prevention of tumor spread and recurrence in several common forms of cancer. Foxy-5 has the formula **For-Met-Asp-Gly-Cys-Glu-Leu-OH** (SEQ_NO 1, Figure 1).

Upon intravenous administration, Foxy-5 binds to and activates WNT5A receptors, predominantly of the Frizzled family, which activates WNT5A-mediated signaling.

Foxy-5 is intended to compensate for the deficiency of protein WNT5A in tumor tissues noted in patients with cancer types where the WNT5A ligand acts as a cancer suppressor, in order to reduce the risk of metastasis. A sub-analysis from a recent retrospective study of patients with colorectal cancer in stage III, shows that the proportion of patients with low expression of WNT5A is significantly higher than that observed in previous studies in patients with stage II colorectal cancer (CRC). Patients with CRC stage III tumors differ from stage II mainly by the presence of tumor cells in lymph nodes adjacent to the primary tumor, thereby being more aggressive and faster progressing. A low level of WNT5A has been observed in close to 70 percent of patients in stage III, compared with approximately 45 percent of patients with less advanced tumor stages. Similar findings have been made in breast cancer tissues, where absence or a low level of WNT5A has been observed in 70% of aggressive triple-negative breast cancer in comparison with approximately 45% in less aggressive breast cancer types. In several types of cancer, including breast-, colon-, prostate- and hepatocellular cancer, it has been demonstrated that a lack or low level of WNT5A in the primary tumor is associated with a more rapid recurrence when compared with tumor with normal or near normal expression of the WNT5A protein. This supports the hypothesis that the WNT5A level significantly influences the course of disease.

Based on a completed Phase 1b study with Foxy-5 aimed at documenting the drug candidate's safety profile, pharmacokinetics and dose determination for Phase 2, Foxy-5 is now undergoing a Phase 2 clinical trial study, the NeoFox study, which includes patients with stage II / III colon cancer, who at diagnosis are considered to have a high risk of recurrence after the primary tumour has been surgically resected. The assessment of the risk of recurrence is performed according to established assessment criteria. The aim of the study is to demonstrate that Foxy-5 reduces the risk of relapse, primarily in patients with low expression of WNT5A. The study is currently underway at 28 hospitals in Spain (16) and Hungary (12).

Foxy-5 itself and a classical solid-phase (SPSS) method for its preparation are described in International Pat. Publication No. WO 2006/130082 (A PEPTIDE LIGAND TO IMPAIR CANCER CELL MIGRATION). The active pharmaceutical ingredient (API) for the preclinical and early clinical studies has been produced by this route. Solution phase routes for Foxy-5 developed by applicant are disclosed in International Patent Publication No. WO 2020/038878 (Solution phase routes for Wnt hexapeptides) and WO 2020/120198 (Linear solution phase synthesis for Wnt hexapeptides).

The current manufacturing process of Foxy-5 applies a product concentration for the final lyophilization of only 3 g/L due to the limited solubility of the salt free drug substance (DS) in water. This presents a bottleneck which severely limits the production throughput for the commercial manufacture of Foxy-5.

Initial attempts at overcoming this issue resulted in the development of an alternative lyophilization process using an aqueous 30% acetonitrile solution (instead of neat water) as solvent. It was demonstrated in small scale experiments and verified on a 35 g scale that the developed process is applicable for DS concentrations up to 7 g/L, *i.e.* a >100% increase in productivity. While this achievement is impressive, it nonetheless remains problematic to produce Foxy-5 on commercial scale when the final step requires such a high dilution.

Consequently, an increase of batch size by other solubilizing techniques than solvent polarity adjustments was proposed. From drug product (DP) formulation studies it was known that a change from the salt free form of Foxy-5 to the sodium salt will increase DS solubility to levels of at least 20 g/L.

It is noted in this context that Foxy-5 contains three carboxylic acid moieties and no free amino groups, as the terminal amino group is formylated (Figure 1). Therefore, it is only possible to prepare salts of Foxy-5 with basic/alkaline compounds. It is noted that some fine chemical suppliers offer a compound marketed as the "trifluoroacetic salt of Foxy-5", which in reality is just a lyophilizate containing free trifluoroacetic acid.

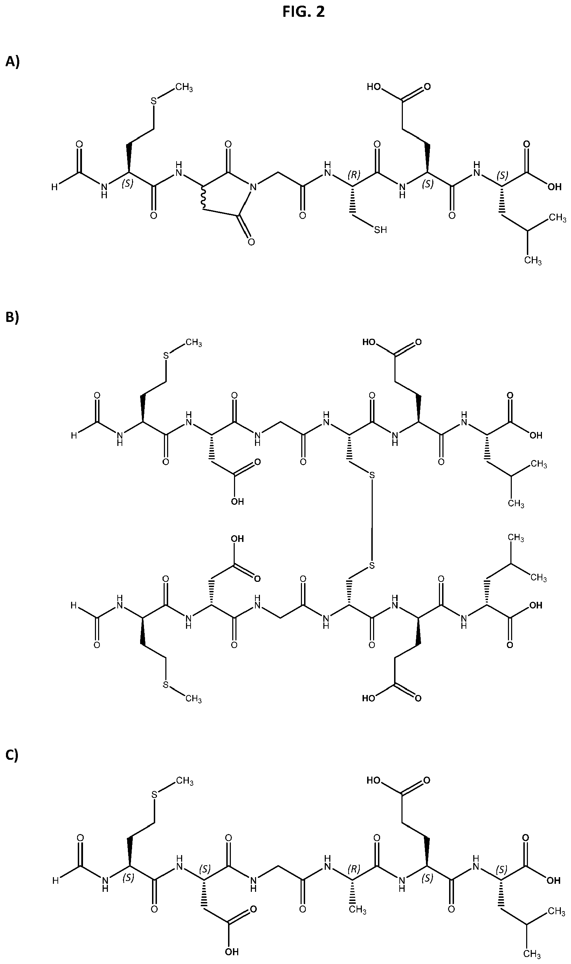

A conversion of Foxy-5 to its sodium salt was investigated by applicant. As Foxy-5 contains three carboxylic groups (Asp², Glu⁵, and the C-terminal carboxylic acids), the mono, di, and tri-sodium salts were generated by treating aqueous slurries of Foxy-5 (25 g/L) with 1 eq., 2 eq. and 3 eq. of sodium hydroxide. As starting material, a batch of Foxy-5 free acid containing very low amounts of two known impurities, the aspartimide (< 0.1%) and the dimer, (< 0.1%) was used (structures, see Figure 2).

The mono-sodium salt of Foxy-5 could not be completely dissolved, and shortly after NaOH addition (1 eq., pH = 3.84), gel formation was observed.

The di-sodium salt could be completely dissolved within 10 minutes after addition of NaOH (2 eq., pH = 4.57). HPLC analysis of the solution showed an increased content of the aspartimide impurity, while dimer formation was only detected on a negligible level. After lyophilization, 0.07% dimer and 0.79% aspartimide were detected.

The tri-sodium salt dissolved within 5 minutes after addition of NaOH (3 eq., pH = 6.76) with minimal increase of the aspartimide impurity, but increased formation of the dimer was observed. After lyophilization, 0.41% dimer and 0.17% of the aspartimide were detected.

Of the three salts, the trisodium salt was found to have the highest solubility, > 100 gr/ltr, and thus initially appeared to be a promising new drug substance candidate.

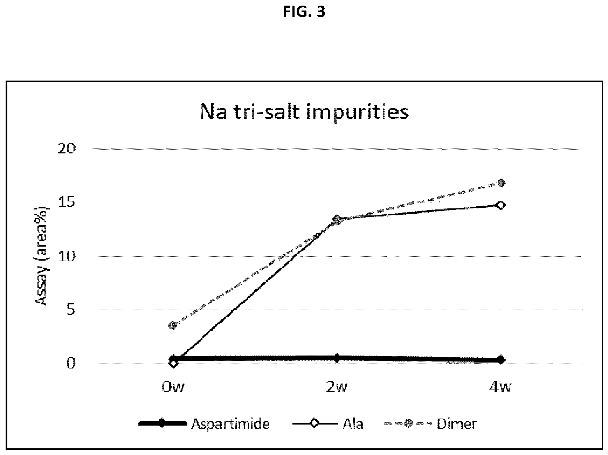

However, upon stability testing at 40°C it was discovered by mass spectrometry that the trisodium salt of Foxy-5 quickly developed significant amounts of the above discussed impurities (Figure 3), reaching a level of between 15-20% after 4 weeks at 40°C.

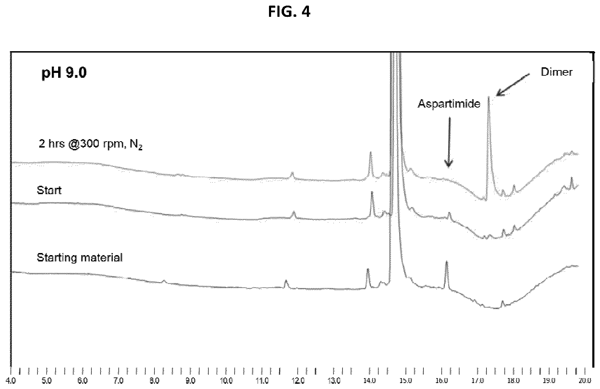

Further studies of aqueous solutions of Foxy-5 at different pH values revealed that at pH 9 the content of the aspartimide significantly decreases directly after complete dissolution. After stirring of the Foxy-5 solution for 2 hours at pH 9 (see Figure 4), the aspartimide peak in the HPLC chromatogram completely disappeared (< LOD). It is known that ring opening of aspartimides occurs at high pH, leading to the formation of either the parent compound and the β-Asp and the D-Asp derivatives. However, significant dimer formation was observed (2.5% after 2 hours at pH = 9). The identification of the dimer in the chromatogram was confirmed by co-elution with separately synthesized reference material. In addition to the two known impurities (the aspartimide and the dimer), a third impurity in the tri-sodium salt was surprisingly identified as the desulfurized Cys → Ala analog of Foxy-5 (see Figure 2 and 3), albeit in low amounts.

In summary, although solubility in water can be increased by converting Foxy-5 to a trisodium salt, this compound is much too unstable to qualify as a drug substance. There thus remains a need for a different DS form of Foxy-5 having satisfactory solubility and stability, both for further clinical trial supply and for future commercial purposes.

### DEFINITIONS

As used herein, the term "nitrogen base" shall mean basic compound comprising at least one nitrogen atom.

As used herein, the term "mono salt" or "mono-salt" shall mean a composition comprising one equivalent of Foxy-5 and 0.5-1.45 equivalents of a nitrogen base, as defined herein. As used herein, the term "di salt" or "di-salt" shall mean a composition comprising one equivalent of Foxy-5 and 1.50 - 2.49 equivalents of a nitrogen base, as defined herein.

As used herein, the term "tri salt" or "tri-salt" shall mean a composition comprising one equivalent of Foxy-5 and 2.5 - 3.1 equivalents of a nitrogen base, as defined herein.

### SUMMARY OF THE INVENTION

Foxy-5 is as mentioned a formylated hexapeptide of formula **For-Met-Asp-Gly-Cys-Glu-Leu-OH** containing three carboxylic acid groups: Asp², Glu⁵, and the C-terminal carboxylic acids groups, and can thus form mono-, di- and tri-salts with basic compounds. It has now been found that Foxy-5 forms highly soluble compositions with various nitrogen bases (defined herein as basic compounds containing at least one nitrogen atom) in the approximate ratios 1:1, 1:2 and 1:3 (Foxy-5 : Base). These compositions are for convenience referred to in the following as mono- di- and tri-salts of Foxy-5, even though the compositions - which are isolated as amorphous lyophilizates - have not been properly (stoichiometrically) characterized as salts.

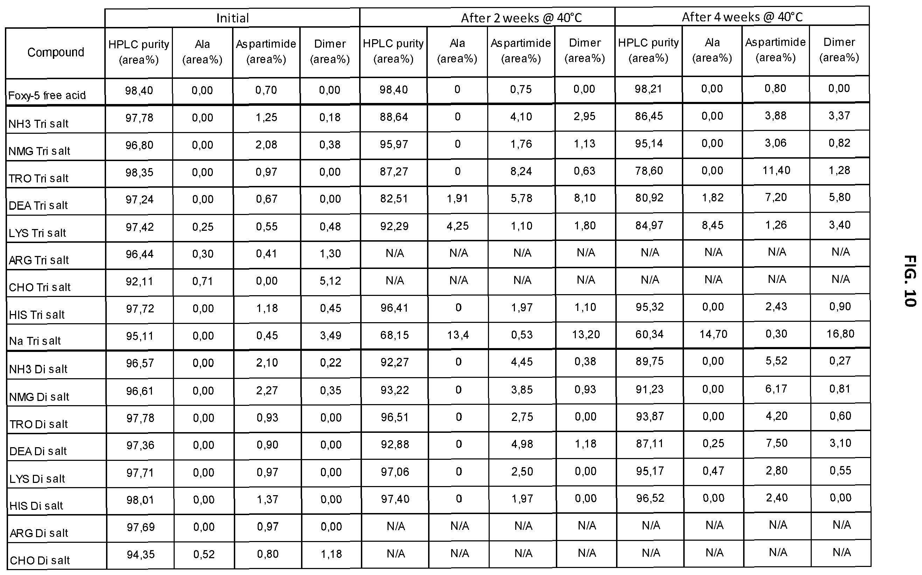

Surprisingly, the stabilities of these compositions when measured at 40 °C have been found to vary considerably, which will be discussed in the following. An overview of all compositions according to the invention and their impurity profiles is given in Figure 10.

**In a first aspect** the present invention provides a solid composition comprising For-Met-Asp-Gly-Cys-Glu-Leu-OH (SEQ_NO 1, Foxy-5) and a nitrogen base selected from ammonia, quaternary ammonium hydroxides, alkylated guanidines, amino acids and primary and secondary amines.

**In a second aspect** the present invention provides a method for producing a composition according to the first aspect, comprising the following steps:
a) Mixing the free acid form of For-Met-Asp-Gly-Cys-Glu-Leu-OH (SEQ_NO 1, Foxy-5), either as
   i. a suspension in a solvent, or
   ii. as a neat powder, with either
   iii. 0.5 - 1.49 equivalents of a nitrogen base to prepare a mono-salt of Foxy-5, or
   iv. 1.50 - 2.49 equivalents of a nitrogen base to prepare a di-salt of Foxy-5, or
   v. 2.5 - 3.1 equivalents of a nitrogen base to prepare a tri-salt of Foxy-5,
b) Stirring the reaction mixture until a clear solution is obtained, and
c) Lyophilizing or spray-drying the solution,

wherein the nitrogen base is a compound selected from ammonia, quaternary ammonium hydroxides, alkylated guanidines, amino acids, and primary and secondary amines, and
wherein all steps a-c are optionally conducted in an inert atmosphere.

### BRIEF DESCRIPTION OF DRAWINGS

**Figure 1**
   The figure shows the chemical structure of Foxy-5 (SEQ_NO 1). Foxy-5 is a linear peptide consisting of six amino acids with a formylated N-terminus. All optically active amino acid residues are in the L-configuration. The molecular formula of Foxy-5 is C₂₆H₄₂N₆O₁₂S₂, and the molecular mass is 694.8 g/mol (average mass).
**Figure 2**
   The figure shows the chemical structure of three main impurities in Foxy-5:
   A) The aspartimide derivative of Foxy-5
   B) The Cys-Cys dimer derivative of Foxy-5
   C) The Cys→ **Ala**-analog of Foxy-5
**Figure 3**
   The figure shows the purity (HPLC area%) values at 0, 2 and 4 weeks storage at 40 °C of three known impurities in the trisodium salt: The "Aspartimide", the "Dimer", and the "Ala-analog". For details, see the Detailed Description.
**Figure 4**
   The figure shows the tracking of high pH treatment of Foxy-5 by HPLC: starting material (bottom trace), after dissolution (middle trace) and after 2 hrs at pH 9 (top trace). The HPLC traces have been offset in the y-direction for purposes of comparison.
**Figure 5**
   The figure shows the purity (HPLC area%) values at 0, 2 and 4 weeks storage at 40°C of the tri-salts (top figure) and the di-salts (bottom) of the invention. As can be seen, there is a greater variability in stability at 4 weeks for the tri-salts than for the di-salts.
**Figure 6**
   The top figure compares the purity (HPLC area%) values at 0, 2 and 4 weeks storage at 40°C of the tri-salts produced with amino acids (i.e. Lys, Arg and His) vs. other salt formers (i.e. choline, diethylamine, ammonia, N-Me-glucamine and tromethamine). The bottom figure shows a similar comparison for the di-salts. As can be seen, there is a general trend that salts produced with amino acids have a higher stability than the salts produced with other salt formers.
**Figure 7**
   The figure shows the purity (HPLC area%) values at 0, 2 and 4 weeks storage at 40 °C of a selection of salts according to the invention having a purity of at least 90% by HPLC (area%) after 4 weeks storage at 40°C.
**Figure 8**
   The figure shows the purity (HPLC area%) values of the freshly lyophilized tri-ammonium (NH3), tri-tromethamine (TRO) and tri-N-methyl-glucamine (NMG) salts of Foxy-5 prepared by either ¹⁾ slow addition of base to an aqueous suspension of Foxy-5, keeping the pH <6 throughout the procedure, or by ²⁾ adding the full amount of base in one portion. As can be seen, the purity of the tri-ammonium salt is quite strongly influenced by the mode of addition, whereas the purity of the tri-TRO and tri-NMG salts is relatively unaffected.
**Figure 9**
   The figure shows the powder X-ray diffractograms (PXRD) of lyophilizates of tri-valent salts of Foxy-5 (bottom to top): Ammonia tri-salt, N-methyl-D-glucamine tri-salt, Tromethamine tri-salt and Histidine tri-salt. The diffractograms have been offset in the y-direction for purposes of comparison.
**Figure 10**
   The figure is an overview table of all conducted experiments resulting in isolated solid compositions according to the invention, divided into "di-salts" and "tri-salts". The table demonstrates the development in impurity profile over time (0, 2 and 4 weeks storage @ 40°C) for each composition and for the starting material (Foxy-5 free acid).

### DETAILED DESCRIPTION

As mentioned in the summary hereinabove a linear solution phase manufacturing method for the Foxy-5 hexapeptide (hereinafter referred to as "Foxy-5") has been published in applicant's international patent application WO 2020/120198 which involves a product concentration of only 3 g/L for the final lyophilization due to the limited solubility of the salt free drug substance (DS) in water. As further mentioned, an initial strategy for overcoming this issue resulted in the production of the tri-sodium salt of Foxy-5, which was found to have a good aqueous solubility but an extremely poor stability which rendered the tri-sodium salt unusable as a new drug substance candidate.

It has now been found that Foxy-5 forms highly soluble compositions with various nitrogen bases. Surprisingly, many of these compositions are significantly more stable than the tri-sodium salt of Foxy-5, which potentially allows for a substantial increase in production throughput for the commercial manufacture of Foxy-5.

This finding was made when performing a screening for salts of Foxy-5 using the following potential "salt formers": ammonium hydroxide, L-arginine, calcium hydroxide, choline hydroxide, diethylamine, L-lysine, L-histidine, magnesium hydroxide, N-methyl-D-glucamine, and tromethamine. In view of the known issues associated with the sodium salts of Foxy-5, this selection of salt formers was believed to provide a sufficient breadth of chemically different candidates which would also eventually satisfy the regulatory requirements for pharmaceutical components and excipients. The selected "salt formers" are thus selected from compounds with GRAS approval and/or Stahl class 1 or 2 designation (see P. H. Stahl and C. G. Wermuth, Handbook of Pharmaceutical Salts: Properties, Selection, and Use, Second Edition, Wiley-VCH: Zürich (2011)).

As mentioned hereinabove the compositions resulting from the salt screening study are for convenience referred to in the following as mono-, di- and tri-salts of Foxy-5, even though the compositions - which are isolated as amorphous lyophilizates - have not been properly (stoichiometrically) characterized as salts.

Tri-salts of Foxy-5 were produced by adding about three equivalents of base to an aqueous suspension of 20-30 mg/mL of the Foxy-5 free acid using two different approaches: adding the calculated amount of base in one portion to the aqueous suspension of the Foxy-5 free acid, or by titrating the relevant base slowly, keeping the pH of the suspension < 6.0 throughout the procedure.

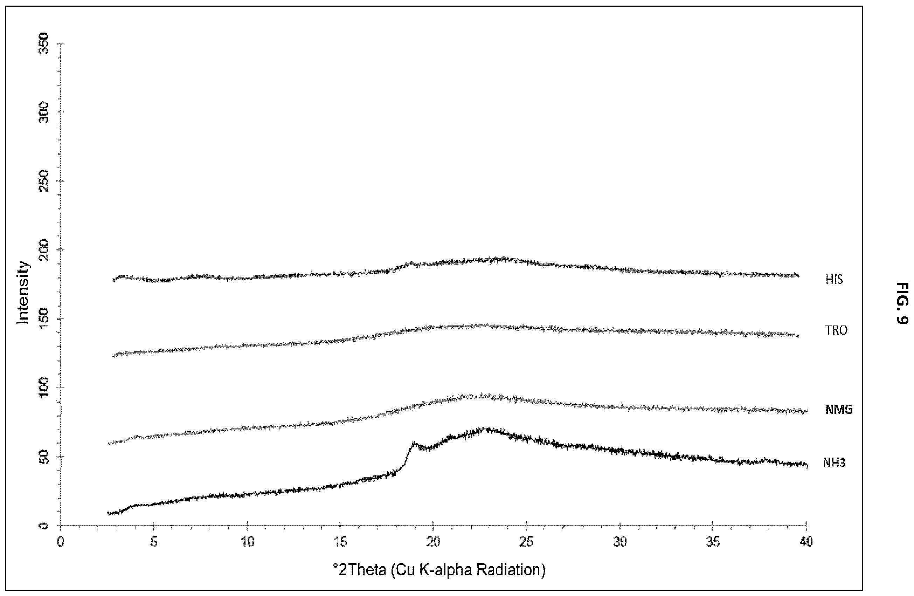

Clear solutions were obtained for all but the calcium and magnesium salts, which remained suspensions, indicating that for all but the calcium and magnesium salts a solubility of at least 20 gr/L was obtained. The clear solutions were lyophilized and the solid lyophilizates characterized by PXRD and HPLC (see Experimental section and Figure 10 for an overview of all compositions). All were found to be amorphous (see Figure 9 for examplary PXRD diagrams). For some salt formers succesful attempts preparing tri-salts with a solubility of ~50 gr/L were made, and for some tri-salts a solubility of >100 gr/L was reached.

The previously studied highly soluble tri-sodium salt (see the Background section) was also generated for comparison.

Di-salts were also prepared with all of the salt formers that led to clear solutions in the trivalent salt experiments, namely, ammonium hydroxide, L-arginine, choline hydroxide, diethylamine, L-lysine, L-histidine, N-methyl-D-glucamine, and tromethamine. Again, clear solutions were obtained in all cases, indicating that also the di-salts of Foxy-5 had a solubility in water that equals or exceeds 20 mg/mL. The solutions were lyophilized and the solid lyophilizates characterized by PXRD and HPLC (see Experimental section). All were found to be amorphous. For some salt formers succesful attempts preparing di-salts with a solubility of ~50 gr/L were made. Mono-salts were prepared in a similar way.

The resulting lyophilizates were all amorphous solids as evidenced by PXRD analysis. In many cases the solids were slightly hygroscopic.

**In a first aspect** the present invention provides a solid composition comprising For-Met-Asp-Gly-Cys-Glu-Leu-OH (SEQ_NO 1, Foxy-5) and a nitrogen base, wherein the nitrogen base is a compound selected from ammonia, quaternary ammonium hydroxides, alkylated guanidines, amino acids, and primary and secondary amines.

In an embodiment of the first aspect, the solid composition contains 0.5 - 3.1 equivalents of a nitrogen base selected from ammonia, quaternary ammonium hydroxides, alkylated guanidines, amino acids, and primary and secondary amines.

In a preferred embodiment of the first aspect, the present invention provides a "tri-salt" comprising Foxy-5 (For-Met-Asp-Gly-Cys-Glu-Leu-OH, SEQ_NO 1) and 2.5 - 3.1 equivalents of a nitrogen base selected from ammonia, quaternary ammonium hydroxides, alkylated guanidines, amino acids, and primary and secondary amines.

In another preferred embodiment of the first aspect, the present invention provides a "di-salt" comprising Foxy-5 (For-Met-Asp-Gly-Cys-Glu-Leu-OH, SEQ_NO 1) and 1.50 - 2.49 equivalents of a nitrogen base selected from ammonia, quaternary ammonium hydroxides, alkylated guanidines, amino acids, and primary and secondary amines.

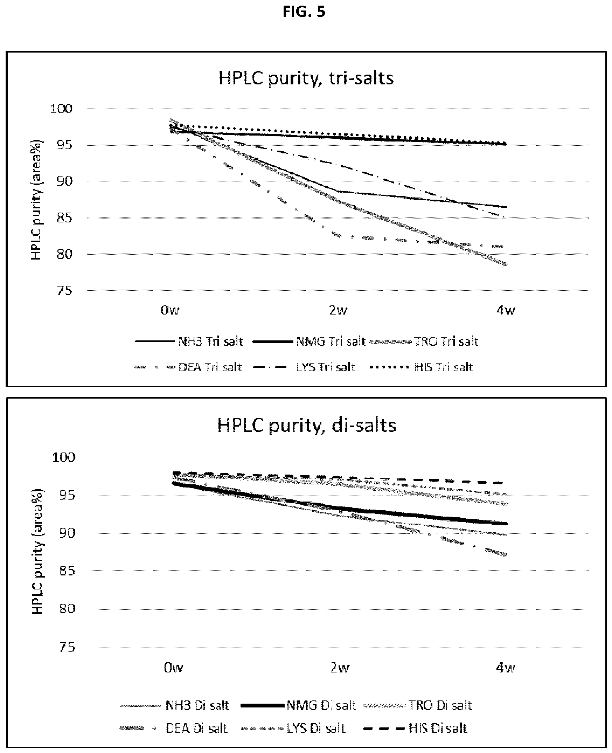

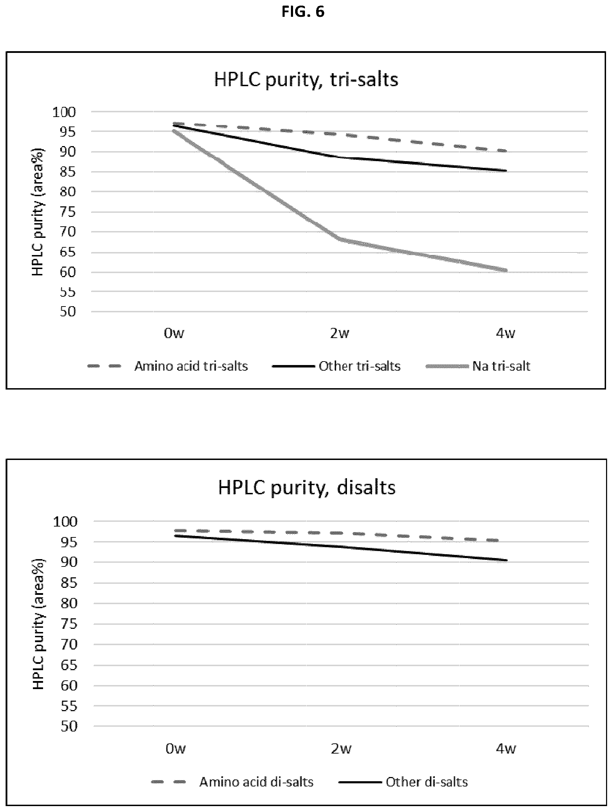

In another embodiment of the first aspect, the present invention provides a "mono-salt" comprising Foxy-5 (For-Met-Asp-Gly-Cys-Glu-Leu-OH, SEQ_NO 1) and 0.5 - 1.49 equivalents of a nitrogen base selected from ammonia, quaternary ammonium hydroxides, alkylated guanidines, amino acids, and primary and secondary amines.
As mentioned in the background section, the tri-sodium salt of Foxy-5 had a high aqueous solubility but was found to be surprisingly unstable in comparison to the free acid form. The major impurities in the sodium salts were found to be (see Figure 2):
- the **Cys** disulfide (dimer) of Foxy 5,
- the **Cys → Ala** substitution, i.e. the desulfurized derivative of Foxy 5, and
- the **Asp** aspartimide derivative.
The stability analysis of the compositions according to the first aspect of the present invention have demonstrated that these compositions contain the same impurities as the known sodium salts, albeit in varying amounts. The development of said impurities over time also varies considerably between the compositions.
A stability study of the compositions according to the first aspect (and the previously prepared trisodium salt) at 40°C for two and four weeks was performed. Some general trends were found. As appears from Figure 5, the absolute purity of the compositions when analysed right after lyophilization (0 weeks) and monitored at 40°C for two and four weeks, was found to differ substantially. The purity of the tri-salts varied more than the purity of the di-salts (HPLC purity 78-95% at 4 weeks for the tri-salts vs. 87-96% at 4 weeks for the di-salts). Furthermore, as appears from Figure 6, the absolute purity of the compositions comprising amino acids was generally higher after 4 weeks at 40 °C when compared with compositions comprising other salt formers (value at 4 weeks: ~90% *vs.* ~85% for tri-salts and ~95% vs. ~90% for di-salts).
In an embodiment the present invention provides a composition comprising Foxy-5 and 2.5 - 3.1 equivalents of an amino acid, such as 2.8 - 3.05 equivalents of an amino acid, such as 2.9 - 3.02 equivalents of an amino acid, such as 2.95 - 3.01 equivalents of an amino acid, such as 3.0 equivalents of an amino acid.
In another embodiment the invention provides a composition comprising Foxy-5 and 1.50 - 2.49 equivalents of an amino acid, such as 1.7 - 2.1 equivalents of an amino acid, such as 1.8 - 2.05 equivalents of an amino acid, such as 1.9 - 2.03 equivalents of an amino acid, such as 1.95 - 2.01 equivalents of an amino acid, such as 2.0 equivalents of an amino acid.

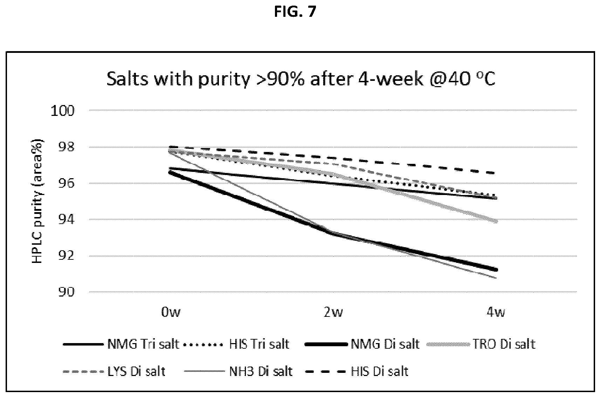

In preferred embodiments the present invention provides a composition comprising Foxy-5 and an amino acid selected from lysine, arginine, or histidine.
Most of the compositions of the present invention are as mentioned hygroscopic to some degree and many were found to turn sticky when subjected to humid air. Assay analysis in combination with elemental analysis has however revealed that some of the compositions of the present invention can be characterized as proper salts which have formed non-sticky hydrates, such as di- and tetrahydrates (see Experimental section). As the compositions of the present invention were isolated as freeze-dried amorphous solids (lyophilizates), such hydrates cannot be as precisely defined as crystalline hydrates, but many of the compositions have nevertheless been found to be quite well-defined such as salts with an approximate ratio of 1:2 ("di-salts") or 1:3 ("tri-salts") between Foxy-5 and the salt-forming base.
Finally, Figure 7 show the compositions having an purity measured by HPLC (area%) of at least 90% after 4 weeks storage at 40 °C. As can be seen, compositions of the present invention with high stability are not limited to a particular salt form.

In another embodiment of the first aspect, the present invention thus provides a composition wherein the nitrogen base is a compound selected from amino acids, ammonia, and primary and secondary amines.

In preferred embodiments of the first aspect, the nitrogen base employed is selected from ammonia, L-lysine, L-histidine, N-methyl-D-glucamine, and tromethamine.

In other preferred embodiments, the present invention provides a composition comprising Foxy-5 and 1.50 - 2.49 equivalents, such as 1.7 - 2.1 equivalents, such as 1.9 - 2.03 equivalents, such as 1.95 - 2.01 equivalents, such as 2.0 equivalents of a nitrogen base selected from ammonia, L-lysine, L-histidine and tromethamine.

In other preferred embodiments, the present invention provides a composition comprising Foxy-5 and 2.5 - 3.1 equivalents, such as 2.9 - 3.03 equivalents, such as 2.95 - 3.01 equivalents, such as 3.0 equivalents, of a nitrogen base selected from L-histidine and N-methyl-D-glucamine.

In other preferred embodiments, the present invention provides a composition comprising the di-ammonium salt, the di-histidine salt, the tri-histidine salt, the di-lysine salt, the diN-methyl-D-glucamine salt, the tri-N-methyl-D-glucamine salt and the di-tromethamine salt of Foxy-5, which salts optionally comprise between 0-5 equivalents of water calculated in relation to Foxy-5.

In a particularly preferred embodiment, the present invention provides a composition comprising Foxy-5 and L-histidine, such as between 1.5 - 3.1 equivalents of L-histidine, such as between 1.7 - 2.49 equivalents of L-histidine, such as 1.7 - 2.1 equivalents of L-histidine or such as between 2.5 - 3.1 equivalents of L-histidine, which compositions optionally comprise between 0-5 equivalents of water calculated in relation to Foxy-5.
**In a second aspect** the present invention provides a method for producing a composition according to the first aspect, comprising the following steps:
a) Mixing the free acid form of For-Met-Asp-Gly-Cys-Glu-Leu-OH (SEQ_NO 1, Foxy-5), either as
   i. a suspension in a solvent, or
   ii. as a neat powder,
      with either
   iii. 0.5 - 1.49 equivalents of a nitrogen base to prepare a mono-salt of Foxy-5, or
   iv. 1.50 - 2.49 equivalents of a nitrogen base to prepare a di-salt of Foxy-5, or
   v. 2.5 - 3.1 equivalents of a nitrogen base to prepare a tri-salt of Foxy-5,
b) Stirring the reaction mixture until a clear solution is obtained, and
c) Lyophilizing or spray-drying the solution,

wherein the nitrogen base is a compound selected from ammonia, quaternary ammonium hydroxides, alkylated guanidines, amino acids, and primary and secondary amines, and
wherein all steps a) - c) are optionally conducted in an inert atmosphere.
In preferred embodiments of the second aspect, the nitrogen base employed for salt formation in step iii), iv) or v) is selected from ammonia, L-lysine, L-histidine, N-methyl-D-glucamine, and tromethamine.
In preferred embodiments, the concentration of Foxy-5 in step i) is about 20-30 mg/mL in water. In other preferred embodiments, the nitrogen base employed for salt formation in step vi) is 1.50 - 2.49 equivalents, such as 1.7 - 2.1 equivalents, such as 1.9 - 2.03 equivalents, such as 1.95 - 2.01 equivalents, such as 2.0 equivalents, of a nitrogen base selected from ammonia, L-lysine, L-histidine and tromethamine.
In other preferred embodiments, the nitrogen base employed for salt formation in step v) is 2.5 - 3.1 equivalents, such as 2.9 - 3.03 equivalents, such as 2.95 - 3.01 equivalents, such as 3.0 equivalents, of a nitrogen base selected from L-histidine and N-methyl-D-glucamine.

As mentioned hereinabove, two different approaches were used in the preparation of the compositions according to the first aspect: adding the calculated amount of base in one portion to the aqueous suspension of the Foxy-5 free acid, or titrating the relevant base slowly, keeping the pH of the reaction mixture < 6.0 throughout the procedure.

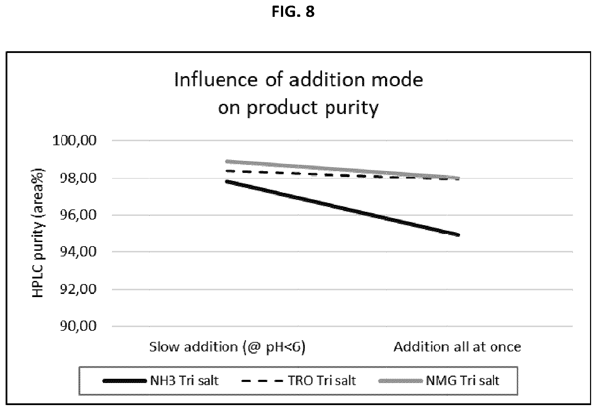

Upon HPLC analysis of the freshly lyophilized compositions, it was found that the "slow" titration produced a composition which contains a lower level of impurities than compositions prepared using the "quick" approach, see Figure 8. As can be seen, the effect is largest for the "tri-salt" of ammonia where the purity drops from about 98% to 95% (slow addition → quick addition). In comparison, the purities of the "tri-salts" of tromethamine and tri-N-methyl-glucamine are only slightly lower when these salts are formed by quick addition. Without being bound by theory, the inventors believe this phenomenon may be related to the pKa value of the salt-forming base: ammonium hydroxide is the strongest of the three bases with a pKa of 9.3, whereas both tromethamine and N-methyl-glucamine have a pKa of 8.0.

In embodiments of the second aspect, the nitrogen base employed for salt formation in step b) is added in one portion to the aqueous suspension of the Foxy-5 free acid. In other embodiments of the second aspect, the nitrogen base employed for salt formation in step b) is added gradually to the aqueous suspension of the Foxy-5 free acid, keeping the pH of the reaction mixture < 6.0 throughout the procedure.

In yet other embodiments of the second aspect, the final clear solution in step 2 is lyophilized to provide an amorphous composition of the present invention.

### EXPERIMENTAL

### Determination of the pKa Values of Foxy-5

The pKa values of the Foxy-5 free acid (lot: 1058263) were measured by potentiometric titration at 25°C using a Sirius T3 instrument equipped with SiriusT3Control Vers. 2.0.0.0 software. An automated aqueous titration method was used with initial values of 1.0 mg of solid API dissolved in 1.5 mL of ISA water and three sequential titrations on the same sample. In all three titrations, the sample solution was adjusted with 0.5 M KOH to a starting pH of 12 and slowly titrated with 0.5 M HCl to a final pH of 2.

Each titration curve was fit using the SiriusT3Refine Vers. 2.0.0.0 software, and the corresponding pKa values were calculated. Values of the pKa for all four theoretically predicted acidic sites were observed in the experimentally accessible range with an RMSD of 0.071. The results of the fit are summarized in below Table 1.

Theoretical pKa values were also calculated with ACD/Percepta 14.51.0 (Build 3382) using both the classic and GALAS modes and the ACD_Prediction Vers. 2.0.0.0 package within the Sirius T3 Vers. 2.0.0.0 software, the latter being the program used to measure the pKa. The assignments of the calculated values to specific sites on the molecule were made by the software. As Table 1 shows, the three prediction methods gave values that agreed with each other to within 0.1 to 0.5 units. The experimental values are reasonably close to the predicted ones with the measured values for the thiol of the cysteine group and the carboxylic acid of the L-aspartic acid lying essentially in the middle of the corresponding predicted ranges and the terminal carboxylic acid and the carboxylic acid of the L-glutamic acid group being just outside the upper end of the predicted range. No concentration errors were present, indicating that the peptide sample was likely quite pure.

**Table 1 Measured and calculated pKₐ values for Foxy-5**

| pKₐ [measured] | ACD Labs ^{a)} | pKₐ [calculated] | | Predicted site (type) |
|---|---|---|---|---|
| | | ACD Labs ^{b)} | Sirius T3 ^{c)} | |
| 9.01 | 9.2 ± 0.1 | 8.7 ± 0.5 | 9.16 | CYS thiol (acidic) |
| 4.79 | 4.4 ± 0.1 | 4.7 ± 0.4 | 4.45 | GLU carboxylic acid (acidic) |
| 3.98 | 4.1 ± 0.1 | 3.9 ± 0.5 | 4.19 | ASP carboxylic acid (acidic) |
| 3.39 | 3.3 ± 0.1 | 3.2 ± 0.4 | 3.34 | terminal carboxylic acid (acidic) |
| a) These values were calculated using the classic approach in ACD/Percepta 14.51.0 (Build 3382). | | | | |
| b) These values were calculated using the GALAS approach in ACD/Percepta 14.51.0 (Build 3382). | | | | |
| c) These values were calculated using the ACD_Prediction Vers. 2.0.0.0 package within the Sirius T3 Vers. 2.0.0.0 software No error bar was provided by the software | | | | |

In the following a detailed description is given for each produced composition according to the present invention, divided into "tri-salts", i.e. Foxy-5 compositions which contain about three equivalents of a nitrogen base, "di-salts", i.e. Foxy-5 compositions which contain about two equivalents of a nitrogen base, and "mono-salts", i.e. Foxy-5 compositions which contain about one equivalent of a nitrogen base.
The stability test data for all formed compositions and the starting material itself (Foxy-5 free acid) were measured at 0, 2 and 4 weeks storage @ 40 °C and included in Figure 10.

### Analytical conditions for stability measurements

The following parameters for the substance-specific UPLC method were employed for the purity measurements of the stability samples:

| **Dionex UltiMate 3000RS UPLC instrument with Chromeleon Version 7.2 software.** | |
|---|---|
| Parameter | Description |
| column (reversed-phase RP18) | Waters, Acquity CSH Phenyl-Hexyl, 150 mm x 2.1 mm, 1.7-µm particles |
| sample dilution system | 87:13 (v/v) H₂O/MeCN |
| mobile phase A | 99.9:0.1 (v/v) 0.1% TFA (v/v) in H2O/MeCN |
| mobile phase B | 0.1% TFA (v/v) in MeCN |
| gradient | 0.0 min - 88% A, 12% B |
| | 1.0 min - 88% A, 12% B |
| | 25.0 min - 74% A, 26% B |
| | 25.5 min - 10% A, 90% B |
| | 28.5 min - 10% A, 90% B |
| | 29.0 min - 88% A, 12% B |
| | 35.0 min - 88% A, 12% B |
| Flow | 0.35 mL min-1 |
| Concentration | approximately 0.1-0.3 mg/mL |
| injection volume | 5 µL |
| detection | 220 nm |
| column temperature | 40°C |
| sample temperature | r.t. |

### Formation of "tri-salts"

Foxy-5 tri-salts are generally prepared by adding the relevant base to a suspension of Foxy-5 in water or another suitable solvent. The base may be added neat or in solution, and either in one portion or gradually, keeping the pH <6 during the procedure.

### Arginine tri-salt

### EXPERIMENT ARG-P1

89.7 mg (0.129 mmol) of Foxy-5 hexapeptide was weighed out into a 4-mL Supelco vial; 3 mL of degassed ultrapure water was added (resistivity ≥ 18.2 MΩ cm) => glassy, white suspension was obtained; 67.7 mg (3 equiv.) of L-arginine was added under an N2 purge => clear, colorless solution with pH 7.31 was obtained; let stir at r.t. for 2 h => clear, colorless solution with pH 7.27 was obtained; filtered through a syringe filter (13-mm diameter, 0.2-µm PVDF) into a 10-mL round-bottomed flask; resulting solution frozen in dry ice; lyophilized for 18 h 15 min => white solid was obtained; characterized by PXRD; stored in the freezer and characterized in duplo by HPLC 14 d later. PXRD confirms that the sample is amorphous; HPLC shows purities of 96.47 area-% and 96.40 area-% for the two injections.

### Calcium tri-salt

### EXPERIMENT Ca-P1

89.6 mg (0.129 mmol) of Foxy-5 hexapeptide was weighed out into a 4-mL Supelco vial; 3 mL of degassed ultrapure water was added (resistivity ≥ 18.2 MΩ cm) => glassy, white suspension was obtained; 28.8 mg (3 equiv.) of Ca(OH)₂ was added => dilute, white suspension with pH 12.36 was obtained; let stir at r.t. for 2 h => dilute, white suspension with pH 12.34 was obtained; let stir at r.t. overnight => white suspension was obtained; experiment terminated because minimum solubility was not reached.

### Choline tri-salt

### EXPERIMENT CHO-P1

89.6 mg (0.129 mmol) of Foxy-5 hexapeptide was weighed out into a 4-mL Supelco vial; 3 mL of degassed ultrapure water was added (resistivity ≥ 18.2 MΩ cm) => white suspension was obtained; 95.4 µL (3 equiv.) of 46% choline hydroxide (aq) (dens. = 1.073 g mL⁻¹) was added under N2 => beige solution with pH 8.51 was obtained; let stir at r.t. for 2 h => beige solution with pH 8.43 was obtained; filtered through a 0.2 µm PVDF syringe filter into a 10-mL round-bottomed flask; solution frozen and lyophilized => beige solid was obtained; characterized by PXRD; characterized in duplo by HPLC. PXRD confirms that the sample is amorphous; HPLC shows purities of 92.14 area-% and 92.08 area-% for the two injections.

### Diethylamine tri-salt

### EXPERIMENT DEA-P1

90.5 mg (0.130 mmol) of Foxy-5 hexapeptide was weighed out into a 4-mL Supelco vial; 3 mL of degassed ultrapure water was added (resistivity ≥ 18.2 MΩ cm) => glassy, white suspension was obtained; 40.2 µL (3 equiv.) of diethylamine (dens. = 0.707 g mL⁻¹) was added under an N2 purge => clear, colorless solution with pH 5.43 was obtained; let stir at r.t. for 2 h => clear, colorless solution with pH 5.45 was obtained; filtered through a syringe filter (13-mm diameter, 0.2 µm PVDF) into a 10-mL round-bottomed flask; resulting solution frozen in dry ice; lyophilized for 18 h 15 min => white solid was obtained; characterized by PXRD; stored in the freezer and characterized in duplo by HPLC 14 d later; aliquots of this sample were stored in closed containers at 40°C for 2 weeks and 4 weeks and examined by HPLC; they were observed to have a glassy appearance after both 2 and 4 weeks of storage at 40°C. PXRD confirms that the sample is amorphous; HPLC shows purities of 97.26 area-% and 97.22 area-% for the two injections.

### Lysine tri-salt

### EXPERIMENT LYS-P1

90.3 mg (0.130 mmol) of Foxy-5 hexapeptide was weighed out into a 4-mL Supelco vial; 3 mL of degassed ultrapure water was added (resistivity ≥ 18.2 MΩ cm) => glassy, white suspension was obtained; 56.8 mg (3 equiv.) of L-lysine was added under an N2 purge => clear, colorless solution with pH 6.39 was obtained; let stir at r.t. for 2 h => clear, colorless solution with pH 6.46 was obtained; filtered through a syringe filter (13-mm diameter, 0.2 µm PVDF) into a 10-mL round-bottomed flask; resulting solution frozen in dry ice; lyophilized for 18 h 15 min => white solid was obtained; characterized by PXRD; stored in the freezer and characterized by HPLC 14 d later; aliquots of this sample were stored in closed containers at 40°C for 2 weeks and 4 weeks and characterized in duplo by HPLC. PXRD confirms that the sample is amorphous; HPLC shows purities of 97.44 area-% and 97.39 area-% for the two injections.

### Magnesium tri-salt

### EXPERIMENT Mg-P1

89.6 mg (0.129 mmol) of Foxy-5 hexapeptide was weighed out into a 4-mL Supelco vial; 3 mL of degassed ultrapure water was added (resistivity ≥ 18.2 MΩ cm) => glassy, white suspension was obtained; 22.7 mg (3 equiv.) of Mg(OH)₂ was added => thick white suspension was obtained (too thick to measure pH); let stir at r.t. for 2 h => white suspension with pH 9.06 was obtained; let stir at r.t. overnight => white suspension was obtained; terminated experiment because minimum solubility not reached. Since no salt with a solubility in excess of 30 g L⁻¹ was obtained, no characterization was carried out.

### Sodium tri-salt

### EXPERIMENT Na-P1

90.5 mg (0.130 mmol) of Foxy-5 hexapeptide was weighed out into a 4-mL Supelco vial; added 3 mL of degassed ultrapure water (resistivity ≥ 18.2 MΩ cm) => glassy, white suspension was obtained; 388.6 µL (3 equiv.) of 1 M NaOH (aq) was added under an N2 purge => clear, colorless solution with pH 8.23 was obtained; let stir at r.t. for 2 h => clear, colorless solution with pH 8.17 was obtained; filtered through a syringe filter (13-mm diameter, 0.45 µm PVDF) into a 10-mL round-bottomed flask; resulting solution frozen in dry ice; lyophilized for 24 h 05 min => white solid was obtained; stored in the freezer and characterized by HPLC 5 d later; the sample was difficult to weigh because it was very hygroscopic; aliquots of this sample were stored in closed containers at 40°C for 2 weeks and 4 weeks and examined in duplo by HPLC; the 4-week sample was difficult to weigh due to hygroscopicity. PXRD confirms that the sample is amorphous; traces of NaCl are possibly present; HPLC shows purities of 95.11 area-% for both injections.

### Ammonia tri-salt

### EXPERIMENT NH3-P8

100.3 mg (0.144 mmol) of Foxy-5 hexapeptide was weighed out into a 15-mL Supelco vial; 3.333 mL of ultrapure water was added (resistivity ≥ 18.2 MΩ cm) under an N2 purge => glassy suspension. The suspension was stirred at 500 rpm and an pH electrode was inserted. 0.1 M of NH₄OH (aq) was dosed at 0.05 mL min-1, pausing to measure pH periodically. At 15 min, 0.50 mL of base had been added and the pH was 3.84; at 45 min, a total of 1.34 mL of base had been added, and the pH was 4.09; at 1 h 06 min, a total of 2.37 mL of base had been added, and the pH was 4.42; at 1 h 14 min, a total of 2.74 mL of base had been added, and the pH was 4.64; a clear, colorless solution was obtained; at 1 h 45 min, a total of 3.74 mL of base had been added (equal to 2.59 equivalents), and the pH was 5.81; the dosing was paused; at 3 h 40 min, the pH was 6.00; the remaining 0.58 mL of base was not added; the experiment was terminated. The clear, colorless solution was placed into a 20-mL round-bottomed flask, frozen in a mixture of dry ice and isopropanol, and lyophilized. HPLC (in duplo) and elemental analyses were carried out. HPLC shows purities of 98.08 area-% and 98.05 area-% for the two injections and corresponding assay values of 68.76% and 68.71%, respectively. The ratio of the nitrogen to sulfur content does not quite comply with a tri-salt, but this is not surprising because the amount added (based on gravimetric calculations) was only 2.59 equivalents. Elemental analysis (C, H, N, O and S) of the product (below) is in good agreement with a salt comprising 2.6 equivalents of ammonia and 2 equivalents of water.

| **CHNOS analysis** | Ammonia "tri-salt" dihydrate C₂₆H₄₂N₅O₄₂S₂ ^{∗} (NH₃)_{2.6} (H₂O)₂ | | | | | | |
|---|---|---|---|---|---|---|---|
| | %C | %H | %N | %O | %S | N/S ratio | Stoichiometry |
| Theoretical | 40,25 | 7,01 | 15,61 | 28,87 | 8,26 | 1,89 | 1 : 2.65 |
| *Found:* | *40,9* | *7,31* | 15,23 | *28,51* | *8,06* | *1,89* | *1 : 2.65* |

### N-methyl-D-glucamine tri-salt

### EXPERIMENT NMG-P5

90.3 mg (0.130 mmol) of Foxy-5 hexapeptide was weighed out into a 7-mL Supelco vial; 3 mL of degassed ultrapure water was added (resistivity ≥ 18.2 MΩ cm). The pH meter was inserted and pH of 2.66 was measured. A N2 purge was applied and stirring was set up at 700 rpm. 0.78 mL of a 0.5 M aqueous solution of N-methyl-D-glucamine (4.8803 g in 50 mL of water) was slowly added with a dosimeter and the pH was measured at different time points. After the addition of 0.25 mL of 0.5 M NMG (aq), a cloudy solution was observed with some material on the bottom of the reactor. pH was 4.01. A clear solution was obtained after the addition of 0.70 mL of the 0.5 M N-methyl-D-glucamine solution; the pH was 5.51. A total of 0.74 mL of 0.5 M N-methyl-D-glucamine solution was added (equal to 2.85 eq); pH was 5.99. After 2h of stirring, the pH of the solution was 6.01. The clear, colorless solution was placed into a 20-mL round-bottomed flask, frozen in dry ice; and lyophilized overnight. A white solid was obtained and characterized by PXRD and HPLC. PXRD confirms that the sample is amorphous; HPLC shows purities of 96.90 area-% and 96.69 area-% for the two injections.

### Tromethamine tri-salt

### EXPERIMENT TRO-P3

90.0 mg (0.1295 mmol) of Foxy-5 hexapeptide was weighed out into a 7-mL Supelco vial; 3 mL of degassed ultrapure water was added (resistivity ≥ 18.2 MΩ cm). The pH meter was inserted and pH of 2.69 was measured. A N2 purge was applied and stirring was set up at 700 rpm. A suspension was observed. 0.39 mL of a 0.5 M aqueous solution of tromethamine (1.2115 g in 20 mL of water) was slowly added with a dosimeter over 30 minutes and the pH was measured at different time points. After the addition of 0.29 mL (1.12 eq) of 0.5 M TRO (aq), almost all the solid was dissolved and pH was 4.04. A clear solution was obtained after the addition of 0.59 mL of the 0.5 M TRO solution (2.28 eq); the pH was 4.99. A total of 0.71 mL of 0.5 M TRO solution was added (equal to 2.74 eq); pH was 5.95. After 2h of stirring, the pH of the solution was 5.97. The clear, colorless solution was placed into a 20-mL round-bottomed flask; frozen in the freezer; and lyophilized overnight. A white solid was obtained and characterized by PXRD. The sample was then stored in the freezer and characterized in duplo by HPLC 5 d later. HPLC shows purities of 98.34 area-% and 98.35 area-% for the two injections.

### Histidine tri-salt

### EXPERIMENT HIS-P2

100.4 mg (0.145 mmol) of Foxy-5 hexapeptide was weighed out into a 7-mL Supelco vial; 3.33 mL of ultrapure water was added (resistivity ≥ 18.2 MΩ cm) under an N2 purge => suspension of white, gel-like solid observed; stirred at 500 rpm and inserted an electrode to measure a pH of 2.64; 0.0985 M of L-histidine (aq) was dosed at 0.1 mL min⁻¹, pausing to measure pH; at 2 min, 0.30 mL of base had been added, and the pH was 3.50; at 9 min, a total of 1.00 mL of base had been added, and the pH was 3.50; at 19 min, a total of 2.00 mL of base had been added, and the pH was 4.23; a mostly clear solution with a few clumps of gel-like solid was observed; at 29 min, a total of 3.00 mL had been added, and the pH was 4.61; at 39 min, a total of 4.00 mL of base had been added, and the pH was 5.11; a clear, colorless solution was observed; at 43 min, a total of 4.38 mL of base had been added, and the pH was 5.29; let stir for several hours; at 3 h 23 min after the start of dosage, the pH was measured to be 5.27, and the experiment was terminated; the clear, colorless solution was placed into a 20-mL round-bottomed flask, resulting solution frozen in dry ice, and lyophilized for 19 h 05 min and yielded a fluffy, white solid; HPLC (in duplo) and elemental analyses were carried out. PXRD confirms that the sample is amorphous (Figure 8). HPLC shows purities of 98.25 area-% and 98.21 area-% for the two injections and corresponding assay values of 62.12% and 61.95%, respectively. Elemental analysis (C, H, N, O and S) of the product (below) is in good agreement with the amorphous product being a tri-salt tetrahydrate.

| **CHNOS analysis** | Histidine tri-salt tetrahydrate C₂₆H₄₂N₆O₁₂S₂ ^{∗} (C₆H₉N₃O₂)₃ (H₂O)₄ | | | | | | |
|---|---|---|---|---|---|---|---|
| | %C | %H | %N | %O | %S | N/S ratio | Stoichiometry |
| Theoretical | 42,93 | 6,29 | 17,17 | 28,46 | 5,14 | 3,28 | 1 : 3.00 |
| *Found:* | *42,89* | *6,48* | *17,19* | 28,27 | *5,16* | *3,33* | *1 : 3.09* |

### Solubility determination for the histidine tri-salt

12.9 mg of the above produced Histidine salt was carefully redissolved in water at a stirring rate of 500 rpm and a water dosing rate of 0.01 mL/min. Complete dissolution was observed after addition of a total of 0.08 mL of water, corresponding to a solubility of at least 161 g/L.

### Formation of "di-salts"

Foxy-5 di-salts are generally prepared by adding the relevant base to a suspension of Foxy-5 in water or another suitable solvent. The base may be added neat or in solution, and either in one portion or gradually, keeping the pH <6 during the procedure.

### Arginine di-salt

### EXPERIMENT ARG-P2

90.5 mg (0.130 mmol) of Foxy-5 hexapeptide was weighed out into a 4-mL Supelco vial; 3 mL of degassed ultrapure water was added (resistivity >_ 18.2 MΩ cm) => glassy, white suspension was obtained; 45.1 mg (2 equiv.) of L-arginine was added under an N2 purge => clear, colorless solution with pH 4.57 was obtained; let stir at r.t. for 2 h => clear, colorless solution with pH 4.55 was obtained; filtered through a syringe filter (13-mm diameter, 0.2-µm PVDF) into a 10-mL round-bottomed flask; resulting solution frozen in dry ice; lyophilized for 19 h 30 min => white solid was obtained; characterized by PXRD; stored in the freezer and characterized in duplo by HPLC 6 d later. PXRD confirms that the sample is amorphous; HPLC shows purities of 97.70 area-% and 97.67 area-% for the two injections.

### Choline di-salt

### EXPERIMENT CHO-P2

89.1 mg (0.128 mmol) of Foxy-5 hexapeptide was weighed out into a 4-mL Supelco vial; 3 mL of degassed ultrapure water was added (resistivity >_ 18.2 MΩ cm) => glassy, white suspension was obtained; 63.6 µL (2 equiv.) of 46% choline hydroxide (aq) (dens. = 1.073 g mL⁻¹) was added under an N2 purge => beige solution with pH 4.78 was obtained; let stir at r.t. for 2 h => beige solution with pH 4.78 was obtained; filtered through a syringe filter (13-mm diameter, 0.2 µm PVDF) into a 10-mL round-bottomed flask; resulting solution frozen in dry ice; lyophilized for 19 h 30 min => white solid was obtained; characterized by PXRD; and characterized in duplo by HPLC. PXRD confirms that the sample is amorphous; HPLC shows purities of 94.42 area-% and 94.27 area-% for the two injections.

### Diethylamine di-salt

### EXPERIMENT DEA-P2

90.1 mg (0.130 mmol) of Foxy-5 hexapeptide was weighed out into a 4-mL Supelco vial; 3 mL of degassed ultrapure water was added (resistivity ≥ 18.2 MΩ cm) => glassy, white suspension was obtained; 26.8 µL (2 equiv.) of diethylamine (dens. = 0.707 g mL⁻¹) was added under an N2 purge => dilute white suspension with pH 4.68 was obtained; let stir at r.t. for 2 h => clear, colorless solution with pH 4.54 was obtained; filtered through a syringe filter (13-mm diameter, 0.2-µm PVDF) into a 10-mL round-bottomed flask; resulting solution frozen in dry ice; lyophilized for 19 h 30 min => white solid was obtained; characterized by PXRD; stored in the freezer and characterized by HPLC 6 d later; aliquots of this sample were stored in closed containers at 40°C for 2 weeks and 4 weeks and examined in duplo by HPLC. PXRD confirms that the sample is amorphous; HPLC shows purities of 97.30 area-% and 97.41 area-% for the two injections.

### Lysine di-salt

### EXPERIMENT LYS-P2

89.6 mg (0.129 mmol) of Foxy-5 hexapeptide was weighed out into a 4-mL Supelco vial; 3 mL of degassed ultrapure water was added (resistivity ≥ 18.2 MΩ cm) => glassy, white suspension was obtained; 37.0 mg (2 equiv.) of L-lysine was added under an N2 purge => clear, colorless solution with pH 4.53 was obtained; let stir at r.t. for 2 h => clear, colorless solution with pH 4.52 was obtained; filtered through a syringe filter (13-mm diameter, 0.2-µm PVDF) into a 10-mL round-bottomed flask; resulting solution frozen in dry ice; lyophilized for 19 h 30 min => white solid was obtained; characterized by PXRD; stored in the freezer and characterized by HPLC 6 d later; aliquots of this sample were stored in closed containers at 40°C for 2 weeks and 4 weeks and examined in duplo by HPLC. PXRD confirms that the sample is amorphous; HPLC shows purities of 97.69 area-% and 97.73 area-% for the two injections.

### Ammonia di-salt

### EXPERIMENT NH3-P6

100.7 mg (0.145 mmol) of Foxy-5 hexapeptide was weighed out into a 7-mL Supelco vial; 3.33 mL of ultrapure water was added (resistivity ≥ 18.2 MΩ cm) under an N2 purge => gel-like, white suspension observed; stirred at 500 rpm and inserted an electrode to measure a pH of 2.63; 1 M of NH₄OH (aq) was dosed at 0.05 mL min⁻¹, pausing to measure pH; at 2 min, 0.10 mL of base had been added, and the pH was 3.19; at 4 min, a total of 0.20 mL of base had been added, and the pH was 3.11; a colorless solution was observed on top of the white suspension; at 5 min, 0.29 mL of base (the di-salt stoichiometry) has been added, and the pH was 3.18; at 7 min, the pH was 5.49, and the solution was nearly clear; at 12 min, the pH was 4.60, and the solution was clear; at 2 h 12 min, the pH was measured to be 4.56, and the experiment was terminated; the clear, colorless solution was placed into a 20-mL round-bottomed flask, resulting solution frozen in dry ice, and stored in the fridge for 5 d; it was then lyophilized for 27 h 35 min and yielded a white solid; HPLC (in duplo) and elemental analyses were carried out. PXRD confirms that the sample is amorphous. HPLC shows purities of 97.62 area-% and 97.66 area-% for the two injections and corresponding assay values of 89.32% and 89.08%, respectively. Elemental analysis (C, H, N, O and S) of the product (below) is in good agreement with the amorphous product being a di-salt dihydrate.

| **CHNOS analysis** | Ammonia di-salt dihydrate C₂₆H₄₂N₆O₁₂S₂ ^{∗} (NH₃)₂ (H₂O)₂ | | | | | | |
|---|---|---|---|---|---|---|---|
| | %C | %H | %N | %O | %S | N/S ratio | Stoichiometry |
| Theoretical | 40,74 | 6,88 | 14,80 | 29,22 | 8,27 | 1,75 | 1: 2.00 |
| *Found:* | *41,41* | *6,97* | *14,21* | *29,35* | *8,06* | *1,76* | *1 : 2.07* |

### N-methyl-D-glucamine di-salt

### EXPERIMENT NMG-P6

90.2 mg (0.130 mmol) of Foxy-5 hexapeptide was weighed out into a 7-mL Supelco vial; 3 mL of degassed ultrapure water was added (resistivity ≥ 18.2 MΩ cm). The pH meter was inserted and pH of 2.67 was measured. A N2 purge was applied to the headspace (N2 atmosphere) and stirring was set up at 700 rpm. A suspension was observed. 0.52 mL of a 0.5 M aqueous solution of N-methyl-D-glucamine (4.8803 g in 50 mL of water) was slowly added with a dosimeter and the pH was measured at different time points. After the addition of 0.35 mL of 0.5 M NMG (aq), almost all the solid was dissolved and a solution with some agglomerates was observed. pH was 4.13. A clear solution was obtained after the addition of 0.50 mL of the 0.5 M N-methyl-D-glucamine solution; the pH was 4.57. A total of 0.52 mL of 0.5 M N methyl-D-glucamine solution was added (correspond to 2.0 eq); pH was 4.64. After 2h of stirring, the pH of the solution was 4.63. The clear, colorless solution was placed into a 20-mL round-bottomed flask; resulting solution frozen in dry ice and lyophilized overnight. A white solid was obtained and characterized by PXRD and HPLC. Aliquots of this sample were stored in closed containers at 40°C for 2 weeks and 4 weeks and examined in duplo by HPLC; they were observed to have a sticky, glassy appearance after 2 weeks of storage at 40°C. PXRD confirms that the sample is amorphous; HPLC shows purities of 96.64 area-% and 96.58 area-% for the two injections.

### Tromethamine di-salt

### EXPERIMENT TRO-P2

90.1 mg (0.130 mmol) of Foxy-5 hexapeptide was weighed out into a 4-mL Supelco vial; 3 mL of degassed ultrapure water was added (resistivity ≥ 18.2 MΩ cm) => glassy, white suspension was obtained; 31.4 mg (2 equiv.) of tromethamine was added under an N2 purge => clear, colorless solution with pH 4.88 was obtained; let stir at r.t. for 2 h => clear, colorless solution with pH 4.86 was obtained; filtered through a syringe filter (13-mm diameter, 0.2-µm PVDF) into a 10-mL round-bottomed flask; resulting solution frozen in dry ice; lyophilized for 19 h 30 min => white solid was obtained; characterized by PXRD; stored in the freezer and characterized in duplo by HPLC 6 d later. PXRD confirms that the sample is amorphous; HPLC shows purities of 97.77 area-% and 97.78 area-% for the two injections.

### Histidine di-salt

### EXPERIMENT HIS-P3

99.2 mg (0.143 mmol) of Foxy-5 hexapeptide was weighed out into a 7-mL Supelco vial; 3.33 mL (i.e. 30 mg/ml) of ultrapure water was added (resistivity ≥ 18.2 MΩ cm) under an N2 purge => suspension of white, gel-like solid observed; stirred at 500 rpm and inserted an electrode to measure a pH of 2.65; 0.0985 M of L-histidine (aq) was dosed at 0.1 mL min⁻¹, pausing to measure pH; at 1 min, 0.17 mL of base had been added, and the pH was 3.10; at 10 min, a total of 1.00 mL of base had been added, and the pH was 3.90; at 20 min, a total of 2.00 mL of base had been added, and the pH was 4.40; a mostly clear solution with a few clumps of gel-like solid was observed; at 28 min, a total of 2.82 mL had been added, and the pH was 4.73; at 1 h 10 min, a clear, colorless solution was observed; at 2 h 28 min after the start of dosage, the pH was measured to be 4.50, and the experiment was terminated; the clear, colorless solution was placed into a 20-mL round-bottomed flask, resulting solution frozen in dry ice, and lyophilized for 19 h 05 min and yielded a fluffy, white solid; HPLC and elemental analyses were carried out; aliquots of this sample were stored in closed containers at 40°C for 2 weeks and 4 weeks and examined by HPLC; the sample was observed to remain a fluffy white solid after 2 and 4 weeks of storage at 40°C. HPLC shows purities of 98.02 area-% and 98.00 area-% for the two injections and corresponding assay values of 67.88% and 67.86%, respectively. Elemental analysis (C, H, N, O and S, below) of the product is in good agreement with the amorphous product being a dii-salt dihydrate.

| **CHNOS analysis** | Histidine di-salt dihydrate C₂₆H₄₂N₆O₁₂S₂ ^{∗} (C₆H₉N₃O₂)₂ (H₂O)₂ | | | | | | |
|---|---|---|---|---|---|---|---|
| | %C | %H | %N | %O | %S | N/S ratio | Stoichiometry |
| Theoretical | 42,37 | 6,36 | 15,60 | 29,71 | 5,95 | 2,62 | 1 : 2.00 |
| *Found:* | *42,98* | *6,67* | *15,15* | *29,43* | *5,77* | *2,63* | *1 : 2.01* |

### Solubility determination for the histidine di-salt

The above experiment was repeated using half the amount of water (60 mg/ml) for the initial slurrying of Foxy-5 hexapeptide. A final concentration of 31.6 g L⁻¹ was achieved for the histidine di-salt at a pH of 5.82. The experiment was also succesfully repeated adding the histidine solution to neat (solid) Foxy-5 hexapeptide which resulted in a final concentration of 49.3 g L⁻¹ at a pH of 5.78. In both cases the final solutions were frozen and lyophilized for 25 hrs yielding a fluffy, white solid.

### Formation of "mono-salts"

Foxy-5 mono-salts are generally prepared by adding the relevant base to a suspension of Foxy-5 in water or another suitable solvent. The base may be added neat or in solution, and either in one portion or gradually, keeping the pH <6 during the procedure. The reaction mixture is then stirred until the starting material has dissolved. The final solution of the mono-salt is optionally filtered followed by being lyophilized or spray-dried.

## Claims

1. A solid composition comprising For-Met-Asp-Gly-Cys-Glu-Leu-OH (SEQ_NO 1, Foxy-5) and a nitrogen base, wherein the nitrogen base is a compound selected from ammonia, quaternary ammonium hydroxides, alkylated guanidines, amino acids, and primary and secondary amines, and wherein said solid composition optionally comprises water.

2. The solid composition according to claim 1 which contains 0.5 - 3.1 molar equivalents of said nitrogen base, calculated in relation to Foxy-5.

3. The solid composition according to any one of claim 1 or 2 wherein the nitrogen base is a compound selected from amino acids, ammonia, and primary and secondary amines.

4. The solid composition according to any one of claim 1 to 3 wherein the nitrogen base is selected from ammonia, L-lysine, L-histidine, N-methyl-D-glucamine, and tromethamine.

5. The solid composition according to any one of claim 1 to 4 comprising Foxy-5 and 1.50 - 2.49 equivalents, such as 1.7 - 2.1 equivalents, such as 1.9 - 2.03 equivalents, such as 1.95 - 2.01 equivalents, such as 2.0 equivalents of a nitrogen base selected from ammonia, L-lysine, L-histidine and tromethamine.

6. The solid composition according to any one of claim 1 to 4 comprising Foxy-5 and 2.5 - 3.1 equivalents, such as 2.9 - 3.03 equivalents, such as 2.95 - 3.01 equivalents, such as 3.0 equivalents, of a nitrogen base selected from L-histidine and N-methyl-D-glucamine.

7. The solid composition according to claim 1 selected from the di-ammonium salt, the di-L-histidine salt, the tri-L-histidine salt, the di-L-lysine salt, the di-N-methyl-D-glucamine salt, the tri-N-methyl-D-glucamine salt and the di-tromethamine salt of Foxy-5, which salts are amorphous and optionally comprise between 0-5 equivalents of water calculated in relation to Foxy-5.

8. The solid composition according to claim 2 wherein the nitrogen base is L-histidine.

9. The solid composition according to claim 8 comprising Foxy-5 and between 1.50 - 2.49 equivalents of L-histidine such as 1.7 - 2.1 equivalents of L-histidine.

10. The solid composition according to claim 8 comprising Foxy-5 and between 2.5 - 3.1 equivalents of L-histidine.

11. A method for producing a solid composition according to any one of the preceding claims, comprising the following steps:
a) Mixing the free acid form of For-Met-Asp-Gly-Cys-Glu-Leu-OH (SEQ_NO 1, Foxy-5), either as
i. a suspension in a solvent, or
ii. as a neat powder,
with either
iii. 0.5 - 1.49 equivalents of a nitrogen base to prepare a monosalt of Foxy-5, or
iv. 1.50 - 2.49 equivalents of a nitrogen base to prepare a disalt of Foxy-5, or
v. 2.5 - 3.1 equivalents of a nitrogen base to prepare a tri-salt of Foxy-5,
b) Stirring the reaction mixture until a clear solution is obtained, and
c) Lyophilizing or spray-drying the solution,
wherein the nitrogen base is a compound selected from ammonia, quaternary ammonium hydroxides, alkylated guanidines, amino acids, and primary and secondary amines, and wherein all steps a) - c) are optionally conducted in an inert atmosphere.

12. A method for producing a solid composition according to claim 11 wherein water is employed as solvent.

13. A method for producing a solid composition according to any one of claim 11-12, wherein the nitrogen base is added gradually to Foxy-5 whilst keeping the pH of the reaction mixture below 6.0.

14. A method for producing a solid composition according to any one of claim 11-12, wherein the resulting clear solution from step b) is lyophilized.

## Patentansprüche

1. Feste Zusammensetzung, umfassend For-Met-Asp-Gly-Cys-Glu-Leu-OH (SEQ_NO 1, Foxy-5) und eine Stickstoffbase, wobei die Stickstoffbase eine Verbindung ausgewählt aus Ammoniak, quaternären Ammoniumhydroxiden, alkylierten Guanidinen, Aminosäuren und primären und sekundären Aminen ist und wobei die feste Zusammensetzung gegebenenfalls Wasser umfasst.

2. Feste Zusammensetzung gemäß Anspruch 1, die 0,5-3,1 Moläquivalente an der Stickstoffbase, berechnet bezogen auf Foxy-5, enthält.

3. Feste Zusammensetzung gemäß einem von Anspruch 1 oder 2, wobei die Stickstoffbase eine Verbindung ausgewählt aus Aminosäuren, Ammoniak und primären und sekundären Aminen ist.

4. Feste Zusammensetzung gemäß einem der Ansprüche 1 bis 3, wobei die Stickstoffbase ausgewählt ist aus Ammoniak, L-Lysin, L-Histidin, N-Methyl-D-glucamin und Tromethamin.

5. Feste Zusammensetzung gemäß einem der Ansprüche 1 bis 4, umfassend Foxy-5 und 1,50-2,49 Äquivalente, wie z.B. 1,7-2,1 Äquivalente, wie z.B. 1,9-2,03 Äquivalente, wie z.B. 1,95-2,01 Äquivalente, wie z.B. 2,0 Äquivalente, an einer Stickstoffbase ausgewählt aus Ammoniak, L-Lysin, L-Histidin und Tromethamin.

6. Feste Zusammensetzung gemäß einem der Ansprüche 1 bis 4, umfassend Foxy-5 und 2,5-3,1 Äquivalente, wie z.B. 2,9-3,03 Äquivalente, wie z.B. 2,95-3,01 Äquivalente, wie z.B. 3,0 Äquivalente, an einer Stickstoffbase ausgewählt aus L-Histidin und N-Methyl-D-glucamin.

7. Feste Zusammensetzung gemäß Anspruch 1, ausgewählt aus dem Diammoniumsalz, dem Di-L-histidinsalz, dem Tri-L-histidinsalz, dem Di-L-lysinsalz, dem Di-N-methyl-D-glucaminsalz, dem Tri-N-methyl-D-glucaminsalz und dem Ditromethaminsalz von Foxy-5, wobei die Salze amorph sind und gegebenenfalls zwischen 0 und 5 Äquivalente Wasser, berechnet bezogen auf Foxy-5, umfassen.

8. Feste Zusammensetzung gemäß Anspruch 2, wobei die Stickstoffbase L-Histidin ist.

9. Feste Zusammensetzung gemäß Anspruch 8, umfassend Foxy-5 und zwischen 1,50 und 2,49 Äquivalente L-Histidin, wie z.B. 1,7-2,1 Äquivalente L-Histidin.

10. Feste Zusammensetzung gemäß Anspruch 8, umfassend Foxy-5 und zwischen 2,5 und 3,1 Äquivalente L-Histidin.

11. Verfahren zur Herstellung einer festen Zusammensetzung gemäß einem der vorstehenden Ansprüche, umfassend die folgenden Schritte:
a) Mischen der freien Säureform von For-Met-Asp-Gly-Cys-Glu-Leu-OH (SEQ_NO 1, Foxy-5) entweder als
i. eine Suspension in einem Lösungsmittel oder
ii. als ein reines Pulver,
mit entweder
iii. 0,5-1,49 Äquivalenten an einer Stickstoffbase, um ein Monosalz von Foxy-5 herzustellen, oder
iv. 1,50-2,49 Äquivalenten an einer Stickstoffbase, um ein Disalz von Foxy-5 herzustellen, oder
v. 2,5-3,1 Äquivalenten an einer Stickstoffbase, um ein Trisalz von Foxy-5 herzustellen,
b) Rühren des Reaktionsgemischs, bis eine klare Lösung erhalten ist, und
c) Lyophilisieren oder Sprühtrocknen der Lösung,
wobei die Stickstoffbase eine Verbindung ausgewählt aus Ammoniak, quaternären Ammoniumhydroxiden, alkylierten Guanidinen, Aminosäuren und primären und sekundären Aminen ist und wobei alle Schritte a)-c) gegebenenfalls in einer inerten Atmosphäre durchgeführt werden.

12. Verfahren zur Herstellung einer festen Zusammensetzung gemäß Anspruch 11, wobei Wasser als Lösungsmittel eingesetzt wird.

13. Verfahren zur Herstellung einer festen Zusammensetzung gemäß einem der Ansprüche 11-12, wobei die Stickstoffbase allmählich zu Foxy-5 zugegeben wird, wobei der pH-Wert des Reaktionsgemischs unter 6,0 gehalten wird.

14. Verfahren zur Herstellung einer festen Zusammensetzung gemäß einem der Ansprüche 11-12, wobei die erhaltene klare Lösung aus Schritt b) lyophilisiert wird.

## Revendications

1. Composition solide comprenant For-Met-Asp-Gly-Cys-Glu-Leu-OH (SEQ_NO 1, Foxy-5) et une base azotée, la base azotée étant un composé choisi parmi l'ammoniac, des hydroxydes d'ammonium quaternaire, des guanidine alkylées, des acides aminés, et des amines primaires et secondaires, et ladite composition solide comprenant éventuellement de l'eau.

2. Composition solide selon la revendication 1 qui contient 0,5 à 3,1 équivalents molaires de ladite base azotée, calculés par rapport à Foxy-5.

3. Composition solide selon l'une quelconque des revendications 1 et 2, la base azotée étant un composé choisi parmi des acides aminés, l'ammoniac, et des amines primaires et secondaires.

4. Composition solide selon l'une quelconque des revendications 1 à 3, la base azotée étant choisie parmi l'ammoniac, la L-lysine, la L-histidine, la N-méthyl-D-glucamine, et la trométhamine.

5. Composition solide selon l'une quelconque des revendications 1 à 4 comprenant Foxy-5 et 1,50 à 2,49 équivalents, tel que 1,7 à 2,1 équivalents, tel que 1,9 à 2,03 équivalents, tel que 1,95 à 2,01 équivalents, tel que 2,0 équivalents d'une base azotée choisie parmi l'ammoniac, la L-lysine, la L-histidine, et la trométhamine.

6. Composition solide selon l'une quelconque des revendications 1 à 4 comprenant Foxy-5 et 2,5 à 3,1 équivalents, tel que 2,9 à 3,03 équivalents, tel que 2,95 à 3,01 équivalents, tel que 3,0 équivalents d'une base azotée choisie parmi la L-histidine et la N-méthyl-D-glucamine.

7. Composition solide selon la revendication 1 choisie parmi le sel de diammonium, le sel de di-L-histidine, le sel de tri-L-histidine, le sel de di-L-lysine, le sel de di-N-méthyl-D-glucamine, le sel de tri-N-méthyl-D-glucamine et le sel de di-trométhamine de Foxy-5, lesquels sels étant amorphes et comprenant éventuellement entre 0 à 5 équivalents d'eau calculés par rapport à Foxy-5.

8. Composition solide selon la revendication 2, la base azotée étant la L-histidine.

9. Composition solide selon la revendication 8 comprenant Foxy-5 et entre 1,50 et 2,49 équivalents de L-histidine tel que 1,7 à 2,1 équivalents de L-histidine.

10. Composition solide selon la revendication 8 comprenant Foxy-5 et entre 2,5 et 3,1 équivalents de L-histidine.

11. Procédé pour la production d'une composition solide selon l'une quelconque des revendications précédentes, comprenant les étapes suivantes :
a) mélange de la forme d'acide libre de For-Met-Asp-Gly-Cys-Glu-Leu-OH (SEQ_NO 1, Foxy-S), soit en tant
i. qu'une suspension dans un solvant, soit
ii. qu'une poudre pure,
avec soit
iii. 0,5 à 1,49 équivalent d'une base azotée pour préparer un monosel de Foxy-5, soit
iv. 1,50 à 2,49 équivalents d'une base azotée pour préparer un disel de Foxy-5, soit
v. 2,5 à 3,1 équivalents d'une base azotée pour préparer un trisel de Foxy-5,
b) agitation du mélange réactionnel jusqu'à ce qu'une solution limpide soit obtenue, et
c) lyophilisation ou séchage par pulvérisation de la solution,
la base azotée étant un composé choisi parmi l'ammoniac, des hydroxydes d'ammonium quaternaire, des guanidine alkylées, des acides aminés, et des amines primaires et secondaires, et toutes les étapes a) à c) étant éventuellement conduites dans une atmosphère inerte.

12. Procédé pour la production d'une composition solide selon la revendication 11, de l'eau étant employée comme solvant.

13. Procédé pour la production d'une composition solide selon l'une quelconque des revendications 11 et 12, la base azotée étant ajoutée graduellement à Foxy-5 tout en maintenant le pH du mélange réactionnel en dessous de 6,0.

14. Procédé pour la production d'une composition solide selon l'une quelconque des revendications 11 et 12, la solution limpide résultante de l'étape b) étant lyophilisée.
